# DEMANDE DE BREVET EUROPEEN

(11) **EP 0 798 007 A1**
(43) Date de publication de la demande: **01.10.1997**
(21) Numéro de dépôt: 97400608.2
(22) Date de dépôt: 19.03.1997
(51) Int. Cl.: A61M 16/00, A61M 11/00, A61B 5/00

(54) **Nébuliseur à capteur de pression**

(30) Priorité: 26.03.1996 FR 9603712; 22.10.1996 FR 9612828
(71) Demandeur: System Assistance Medical, 47300 Villeneuve-sur-Lot (FR)
(72) Inventeur: Cinquin, Gérard, 47300 Villeneuve-sur-Lot (FR)
(74) Mandataire: Casalonga, Axel

(57) **Abrégé**

Nébuliseur comprenant des moyens (5) pour former un brouillard à partir d'un liquide et des moyens pour transporter ce brouillard vers un patient, dans lequel ces moyens de transport comprennent un canal de circulation d'air comprenant une entrée d'air et une sortie vers le patient, entre lesquelles le brouillard formé est pris par le courant d'air, ainsi qu'une sortie à l'air libre, le nébuliseur comprenant en outre une valve d'entrée anti-retour (31) disposée dans ladite entrée d'air, une valve de sortie anti-retour (32) disposée dans ladite sortie à l'air libre (30), des moyens (36, 37) de prise de la pression dans lesdits moyens de transport et des moyens (39) pour délivrer un signal de signalisation ou d'avertissement fonction de la valeur du signal fourni par lesdits moyens de prise de pression et déterminer au moins un seuil de niveau du signal de signalisation ou d'avertissement.

## Description

La présente invention concerne un nébuliseur permettant de délivrer à un patient un brouillard par exemple contenant un médicament en vue d'un traitement en particulier de ses voies respiratoires.

Le nébuliseur selon l'invention comprend des moyens pour former un brouillard à partir d'un liquide et des moyens pour transporter ce brouillard vers un patient.

Selon l'invention, lesdits moyens de transport comprennent un canal de circulation d'air comprenant une entrée d'air et une sortie vers le patient, entre lesquelles le brouillard formé est pris par le courant d'air, ainsi qu'une sortie à l'air libre.

Le nébuliseur selon l'invention comprend en outre une valve d'entrée anti-retour disposée dans ladite entrée d'air, une valve de sortie anti-retour disposée dans ladite sortie à l'air libre, des moyens de prise de la pression dans lesdits moyens de transport et des moyens pour délivrer un signal de signalisation ou d'avertissement en fonction de la valeur du signal fourni par lesdits moyens de prise de pression et déterminer au moins un seuil de niveau du signal de signalisation ou d'avertissement.

Ainsi, le patient peut contrôler le débit du courant d'air qu'il provoque dans ledit canal de circulation lorsqu'il inspire, en constatant l'amplitude ou la valeur dudit signal de signalisation ou d'avertissement par rapport audit seuil de niveau correspondant à une dépression déterminée dans ledit canal de circulation d'air et ainsi tendre à inhaler des quantités de brouillard prédéterminées, fonction en particulier dudit seuil de niveau.

Selon une variante de l'invention, lesdits moyens de transport comprennent un conduit d'entrée d'air comprenant ladite valve d'entrée anti-retour, une chambre dans laquelle débouche ledit conduit d'entrée, un conduit d'amenée pour relier ladite chambre au patient, ainsi qu'un conduit de sortie à l'air libre branché sur ledit conduit d'amenée et comprenant ladite valve de sortie anti-retour, le brouillard se formant dans ladite chambre.

Selon l'invention, ledit conduit d'amenée comprend de préférence un conduit en T dont la branche principale est reliée à ladite chambre et les deux autres branches sont l'une reliée au patient et l'autre à l'air libre, cette branche reliée à l'air libre comprenant ladite valve de sortie anti-retour.

Selon une autre variante de l'invention, lesdits moyens de transport comprennent un conduit d'entrée d'air comprenant ladite valve d'entrée anti-retour, un conduit d'amenée vers le patient, un conduit de sortie à l'air libre comprenant ladite valve de sortie anti-retour, ainsi qu'un conduit d'accès branché entre ledit conduit d'entrée et ledit conduit d'amenée et relié à une chambre dans laquelle se forme le brouillard.

Selon l'invention, ledit conduit de sortie à l'air libre est de préférence branché sur ledit conduit d'amenée, en aval du branchement précité dudit conduit d'accès.

Selon l'invention, lesdits moyens de prise de pression sont de préférence disposés en amont de la valve d'entrée anti-retour dudit conduit d'entrée.

Selon l'invention, lesdits moyens de prise de pression sont de préférence disposés en amont du branchement précité dudit conduit d'accès.

Selon l'invention, ladite chambre peut avantageusement être formée dans une cuve fermée par un couvercle sur lequel sont branchés lesdits conduits d'entrée et d'amenée.

Selon l'invention, ladite chambre peut avantageusement être formée dans une cuve munie d'un couvercle sur lequel est branché ledit conduit d'accès.

Selon l'invention, lesdits moyens de prise de pression peuvent avantageusement comprendre une tubulure branchée sur lesdits moyens de transport, au fond de laquelle est disposé un capteur de pression.

Selon l'invention, le nébuliseur comprend de préférence une coupelle disposée dans une cuve et fermée par un couvercle sur lequel sont branchés lesdits conduits d'entrée et d'amenée.

Selon l'invention, lesdits moyens pour délivrer un signal de signalisation ou d'avertissement peuvent avantageusement comprendre deux seuils de niveau du signal de signalisation ou d'avertissement.

Selon l'invention, lesdits moyens pour délivrer un signal de signalisation ou d'avertissement peuvent avantageusement comprendre des moyens visuels.

Selon l'invention, lesdits moyens pour délivrer un signal de signalisation ou d'avertissement peuvent avantageusement comprendre des diodes électroluminescentes d'au moins deux couleurs permettant de distinguer ledit seuil de niveau et des moyens pour alimenter sélectivement ces diodes en fonction de la valeur du signal fourni par lesdits moyens de prise de pression.

Selon l'invention, le niveau des signaux desdits moyens de signalisation ou d'avertissement est de préférence réglable.

La présente invention sera mieux comprise à l'étude d'un nébuliseur décrit à titre d'exemple non limitatif et illustré par le dessin sur lequel :
- la figure 1 représente une coupe verticale d'un nébuliseur selon la présente invention ;
- la figure 2 représente le schéma d'un circuit électronique pour le fonctionnement de ce nébuliseur
- la figure 3 représente une coupe verticale d'un autre nébuliseur selon la présente invention ;
- et la figure 4 représente une coupe verticale d'un autre nébuliseur selon la présente invention.

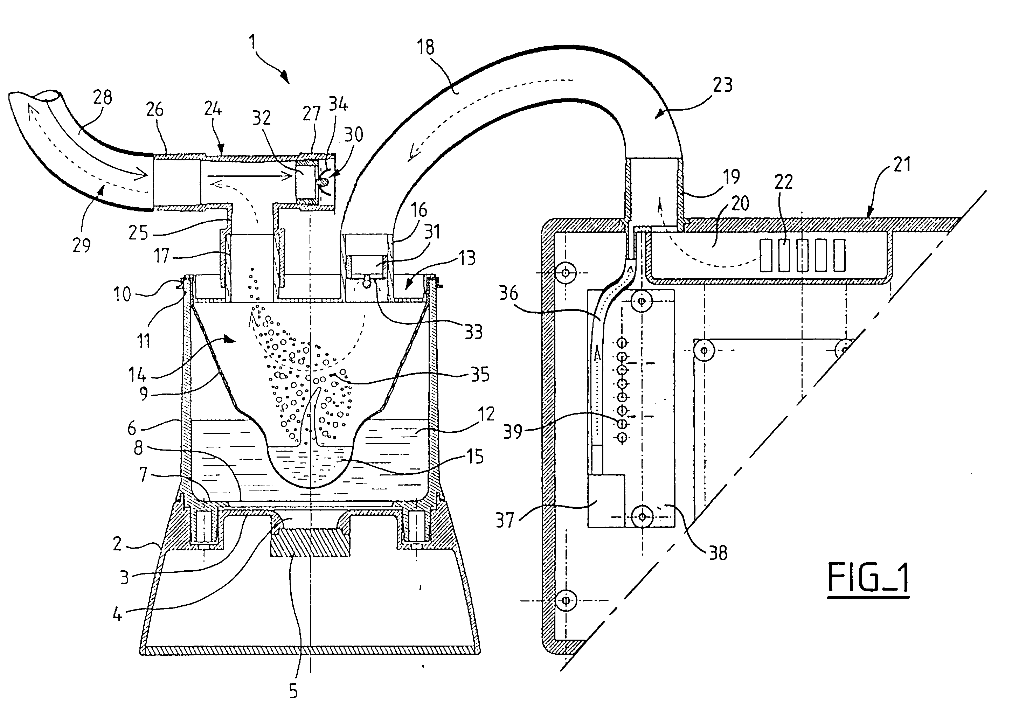

En se reportant à la figure 1, on voit qu'on a représenté un nébuliseur repéré d'une manière générale par la référence 1, qui comprend un socle 2 qui présente une paroi horizontale supérieure 3 au milieu de laquelle est prévu un passage 4 dans la partie inférieure duquel est fixé de façon étanche un émetteur d'ondes à quartz 5.

Le nébuliseur 1 comprend une cuve cylindrique 6 qui est montée sur la paroi supérieure 3 du socle 2 et dont le fond 7 présente une ouverture 8 de communication avec le passage 4.

Dans la cuve 6 est disposée une coupelle 9 dont le bord périphérique supérieur 10 est porté par le bord périphérique supérieur 11 de la cuve 6, la partie inférieure de cette coupelle étant à distance de la face supérieure du quartz 5 et la cuve 6 étant partiellement remplie d'un liquide tel que de l'eau 12, de telle sorte que la partie inférieure de la coupelle 9 plonge dans ce liquide 12.

Un couvercle horizontal 13 est engagé dans la partie supérieure de la coupelle 9 et délimite avec cette dernière une chambre de nébulisation 14 dans laquelle un liquide 15 peut être transformé en un brouillard de fines goutellettes sous l'effet du quartz 5.

Le couvercle 13 présente, en saillie vers le haut, une tubulure d'entrée 16 et une tubulure de sortie 17.

La tubulure d'entrée 16 du couvercle 13 est reliée, par l'intermédiaire d'un tuyau souple 18 à une tubulure de sortie 19 d'une chambre 20 ménagée dans un boîtier annexe 21, cette chambre 20 étant reliée à l'air libre par l'intermédiaire d'ouvertures 22 ménagées dans sa paroi. La chambre 20, la tubulure 19, le tuyau 18 et la tubulure 16 constituent un conduit d'entrée 23 reliant l'air libre à la chambre de nébulisation 14.

Sur la tubulure de sortie 17 du couvercle 13 est montée une pièce tubulaire en T 24 dont la branche principale 25 est portée par la tubulure 17 et dont les branches opposées 26 et 27 sont la première reliée à un patient par l'intermédiaire d'un tuyau souple 28 et la seconde reliée à l'air libre. La tubulure 17, le T de dérivation 24 et le tuyau 28 constituent un conduit d'amenée 29 sur lequel est branché un conduit de sortie 30 constitué par la branche 27 du T de dérivation 24.

Dans la tubulure d'entrée 16 du couvercle 13 est disposée une valve d'entrée anti-retour 31 qui n'autorise le passage d'air qu'en direction de la chambre de nébulisation 14.

Dans la branche 27 du T de dérivation 24 est disposée une valve de sortie anti-retour 32 qui n'autorise le passage d'air qu'en direction de l'extérieur.

Dans l'exemple représenté, les valves anti-retour 31 et 32 comprennent des membranes élastiques radiales 33 et 34 qui, en position statique, obstruent respectivement les conduits 23 et 30 et qui, sous l'effet d'un courant d'air, se déforment élastiquement à partir de cette position pour laisser passer l'air.

Ainsi, lorsque le patient aspire par l'extrémité libre du conduit d'amenée 28, la valve d'entrée anti-retour 31 est ouverte et la valve de sortie anti-retour 32 est fermée. Lors de cette inspiration, le patient provoque un courant d'air dans le canal d'écoulement formé par le conduit d'entrée 23, la chambre 14 et le conduit d'amenée 29 et inhale le brouillard 35 qui se forme en continu dans la chambre de nébulisation 14 sous l'effet du quartz 5.

A l'opposé, lorsque le patient expire par le tuyau 28, la valve d'entrée anti-retour 31 est fermée et la valve de sortie anti-retour 32 est ouverte. L'air expiré par le patient sort vers l'extérieur par le conduit de sortie à l'air libre 30. Compte tenu du T de dérivation 24, cette expiration ne produit pas ou peu d'évacuation vers l'extérieur du brouillard 35 qui continue de se former dans la chambre de nébulisation 14.

Par ailleurs, le nébuliseur 1 comprend un tuyau souple 36 qui débouche latéralement dans la tubulure 19 du conduit d'amenée 23 et qui présente une section faible par rapport à celle du conduit 23, par exemple inférieure à son dixième. Ce tuyau souple 36, qui s'étend dans le boîtier 21 est connecté à un capteur de pression 37 porté par une plaque de circuit imprimé 38. Cette plaque 38 porte en outre, dans l'exemple, huit diodes électroluminescentes ou leds 39 disposées les unes au-dessus des autres et visibles de l'extérieur du boîtier 21.

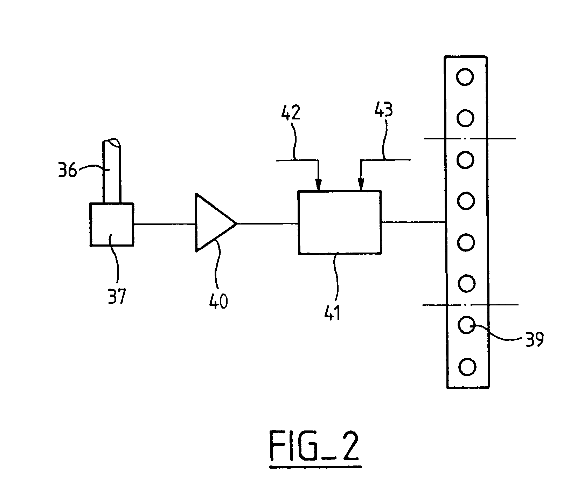

En se reportant à la figure 2, on voit que le nébuliseur 1 comprend un circuit électronique, par exemple porté par la plaque 38, qui comprend un amplificateur 40 qui reçoit les signaux de sortie du capteur de pression 37 et qui délivre des signaux amplifiés à un sélecteur 41 d'alimentation des diodes électroluminescentes 39.

Ce sélecteur d'alimentation 41 est tel qu'il assure un allumage successif et progressif des diodes électroluminescentes 39 en fonction de la valeur du signal fourni par le capteur de pression 37, de préférence proportionnellement à la dépression dans le conduit d'entrée 23.

Le sélecteur 41 présente une entrée de réglage 42 permettant de régler une valeur inférieure d'allumage pour laquelle la première diode électroluminescente s'allume et une entrée de réglage 43 permettant de régler une valeur supérieure d'allumage pour laquelle la huitième diode électroluminescente s'allume après allumage successif des autres.

De plus, dans un exemple, les deux diodes électroluminescentes s'allumant les premières peuvent être de couleur rouge, les quatre suivantes de couleur verte et les deux dernières de couleur rouge. Ainsi, le nébuliseur 1 permet de visualiser deux seuils de niveau pour le signal de sortie du capteur 37, qui sont situés entre les deuxième et troisième diodes et entre les sixième et septième diodes.

Lorsque le patient est en phase d'aspiration comme décrit précédemment, la dépression qui s'établit dans le conduit d'entrée 23 se trouve mesurée par le capteur 37 et le patient constate l'amplitude de cette dépression en fonction du nombre de diodes électroluminescentes 39 allumées.

En réglant comme souhaité le sélecteur 41 par ses entrées de réglage 42 et 43, on peut donc imposer un mode d'aspiration au patient en lui assignant la consigne d'aspirer de telle sorte quc son aspiration permette d'allumer au moins les deux premières diodes électroluminescentes sans pour autant que les deux dernières diodes soient allumées.

Grâce aux dispositions qui précèdent, le nébuliseur 1 permet de façon simple de prescrire au patient des quantités de brouillard 35 instantanément prédéterminées en fonction approximativement d'un débit imposé d'air dans le conduit d'entrée 23. Les quantités inhalées par le patient dépendent en outre de la durée de chaque inspiration, de la durée entre chacune de ses inspirations et du réglage du quartz 5 qui détermine la quantité de brouillard qui se forme en continu dans la chambre 14.

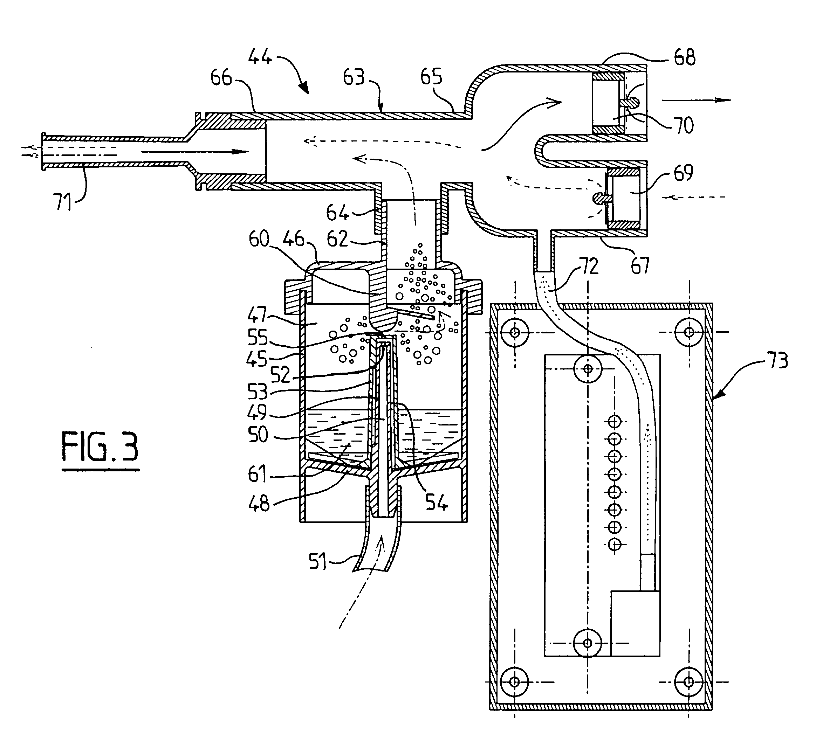

En se reportant à la figure 3, on voit qu'on a représenté un autre nébuliseur, repéré d'une manière générale par la référence 44, qui se différencie du nébuliseur 1 par ses moyens pour former un brouillard à partir d'un liquide et ses moyens pour transporter ce brouillard vers un patient.

Le nébuliseur 44 comprend une cuve 45 muni d'un couvercle 46, qui délimite une chambre de nébulisation 47. Le fond 48 de la cuve 45 est muni, en son milieu, d'une colonne 49 qui s'étend vers le haut et qui présente un canal central 50 relié, en-dessous du fond 49, à un tuyau souple 51 d'amenée d'air sous pression, l'extrémité supérieure de la colonne 49 présentant un orifice 52 de sortie d'air.

Sur la colonne 49 est disposé un manchon 53 qui délimite un canal 54 qui communique avec la partie inférieure de la cuve 45, la partie supérieure du manchon 53 présentant un orifice de sortie 55 situé en face et à faible distance de l'extrémité inférieure d'une partie en saillie vers le bas 60 du couvercle 46.

Ainsi, l'air amené par le canal inférieur 50 de la colonne 49 provoque une aspiration du liquide 61 introduit dans la cuve 45 pour le projeter au travers de l'orifice de sortie 55 du manchon 53 contre l'extrémité inférieure de la partie en saillie 60, cette projection provoquant la formation d'un brouillard constitué de très fines gouttelettes de liquide.

Lc couvercle 46 présente une embouchure verticale 62 au travers de laquelle le brouillard formé est évacué.

Le nébuliseur 44 comprend en outre une pièce tubulaire 63 en forme de T qui comprend une branche centrale 64 et deux branches opposées 65 et 66.

La branche centrale 64 est branchée sur l'embouchure 62 du couvercle 46 de manière à constituer une conduite d'accès pour le brouillard qui se forme dans la chambre 47. La branche 65 est divisée en deux de manière à constituer une conduite d'entrée d'air 67 et une conduite de sortie à l'air libre 68, dans les extrémités desquelles sont montées des valves anti-retour 69 et 70 équivalentes aux valves 31 et 32 de l'exemple précédent. La branche 66 constitue une conduite d'amenée à l'extrémité de laquelle est branché un embout buccal 71 qui pourrait être remplacé par un tuyau souple d'amenée vers un embout buccal éloigné.

Ainsi, lorsque le patient aspire par l'extrémité de l'embout buccal 71, la valve d'entrée anti-retour 69 est ouverte et la valve de sortie anti-retour 70 est fermée. Lors de cette aspiration, le patient provoque un courant d'air dans le canal d'écoulement formé par le conduit d'entrée 67, le conduit d'amenée 66 et l'embout buccal 71, ce courant d'air prenant, lors de son passage dans la zone de liaison du conduit d'accès 64, le brouillard qui se forme en continu dans la chambre 47 pour l'entraîner jusqu'au patient.

A l'opposé, lorsque le patient expire par l'extrémité de l'embout buccal 71, la valve d'entrée anti-retour 69 est fermée et la valve de sortie anti-retour 70 est ouverte. L'air expiré par le patient sort vers l'extérieur par le conduit de sortie à l'air libre 68.

Par ailleurs, le nébuliseur 44 comprend un tuyau souple 72 qui débouche latéralement dans le conduit d'entrée 67, en aval de la valve anti-retour 69, et qui présente une section faible par rapport à celle de ce conduit 67.

Comme dans l'exemple précédent, ce tuyau souple 72 constitue une prise de pression et est relié à un boîtier électronique 73 correspondant au boîtier électronique 21 du nébuliseur décrit en référence aux figures 1 et 2 et fonctionnant de la même manière.

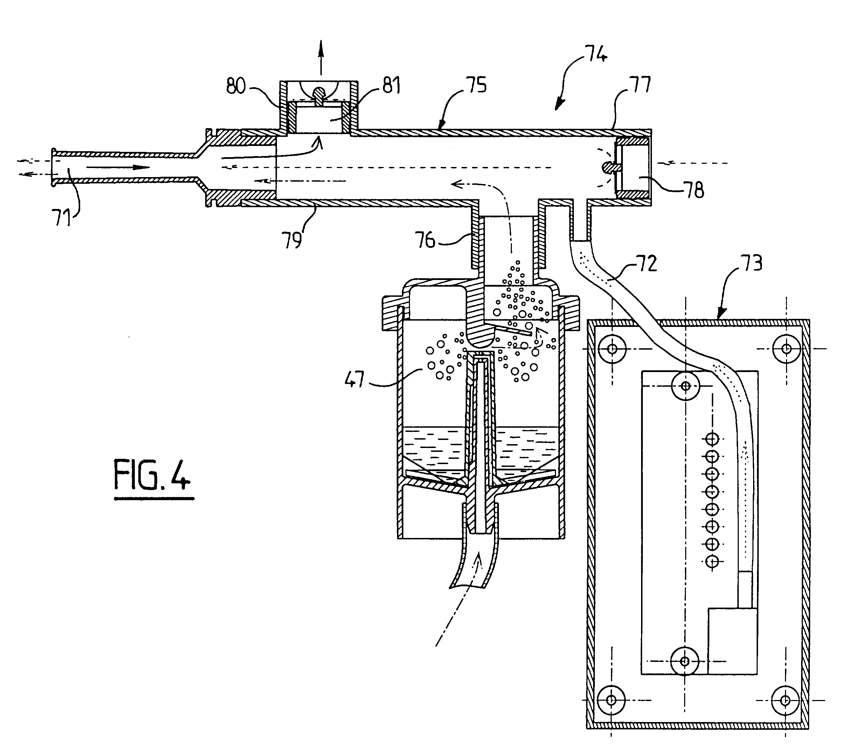

En se reportant maintenant à la figure 4, on voit qu'on a représenté un nébuliseur, repéré d'une manière générale par la référence 74, qui constitue une variante d'exécution du nébuliseur 44 décrit en référence à la figure 3 et qui s'en différencie par sa pièce tubulaire 75 en forme de T.

Cette pièce tubulaire 75 comprend une branche centrale 76 montée sur la tubulure 62 pour être reliée à la chambre de nébulisation 47 de l'exemple précédent ainsi qu'à l'opposé l'une de l'autre, d'une part un conduit d'entrée 77 dans l'extrémité duquel est disposée une valve anti-retour 78 et d'autre part un conduit d'amenée 79 sur lequel est branché latéralement, en aval de la zone de liaison du conduit d'accès 76, un conduit de sortie à l'air libre 80 dans l'extrémité duquel est disposée une valve anti-retour 81, l'extrémité de la branche 79 portant l'embout buccal 71 de l'exemple précédent.

Dans cette variante, lorsque le patient expire, le courant d'air d'expiration ne passe pas devant le conduit d'accès 76.

Par ailleurs, le tuyau souple de prise de pression 72 de l'exemple précédent est branché sur le conduit d'entrée 77, en aval de la valve anti-retour 78 et en amont de la zone de liaison du conduit d'accès 76 entre le conduit d'entrée 77 et le conduit d'amenée 79.

La présente invention ne se limite pas aux exemples qui viennent d'être décrits. En particulier, les moyens de prise de pression pourraient être à un autre endroit des moyens de transport du brouillard et les valves anti-retour pourraient être de structure différente. En outre, les moyens produisant le brouillard pourraient être différents.

## Revendications

1. Nébuliseur comprenant des moyens (5) pour former un brouillard à partir d'un liquide et des moyens pour transporter ce brouillard vers un patient, caractérisé par le fait que ces moyens de transport comprennent un canal de circulation d'air comprenant une entrée d'air et une sortie vers le patient, entre lesquelles le brouillard formé est pris par le courant d'air, ainsi qu'une sortie à l'air libre, et qu'il comprend en outre une valve d'entrée anti-retour (31) disposée dans ladite entrée d'air, une valve de sortie anti-retour (32) disposée dans ladite sortie à l'air libre (30), des moyens (36, 37) de prise de la pression dans lesdits moyens de transport et des moyens (39) pour délivrer un signal de signalisation ou d'avertissement fonction de la valeur du signal fourni par lesdits moyens de prise de pression et déterminer au moins un seuil de niveau du signal de signalisation ou d'avertissement, de telle sorte que le patient peut contrôler le débit du courant d'air qu'il provoque dans ledit canal de circulation lorsqu'il inspire, en constatant l'amplitude ou la valeur dudit signal de signalisation ou d'avertissement par rapport audit seuil de niveau correspondant à une dépression déterminée dans ledit canal de circulation d'air et ainsi tendre à inhaler des quantités de brouillard prédéterminées, fonction en particulier dudit seuil de niveau.

2. Nébuliseur selon la revendication 1, caractérisé par le fait que lesdits moyens de transport comprennent un conduit d'entrée d'air (23) comprenant ladite valve d'entrée anti-retour (31), une chambre (14) dans laquelle débouche ledit conduit d'entrée, un conduit d'amenée (29) pour relier ladite chambre au patient, ainsi qu'un conduit de sortie à l'air libre branché sur ledit conduit d'amenée et comprenant ladite valve de sortie anti-retour (32), le brouillard se formant dans ladite chambre.

3. Nébuliseur selon la revendication 2, caractérisé par le fait que ledit conduit d'amenée (29) comprend un conduit en T (24) dont la branche principale (25) est reliée à ladite chambre (14) et les deux autres branches sont l'une (26) reliée au patient et l'autre (27) à l'air libre, cette branche reliée à l'air libre comprenant ladite valve de sortie anti-retour (32).

4. Nébuliseur selon la revendication 1, caractérisé par le fait que lesdits moyens de transport comprennent un conduit d'entrée d'air (67) comprenant ladite valve d'entrée anti-retour (69), un conduit d'amenée (66) vers le patient, un conduit de sortie (68) à l'air libre comprenant ladite valve de sortie anti-retour (70), ainsi qu'un conduit d'accès (64) branché entre ledit conduit d'entrée et ledit conduit d'amenée et relié à une chambre (47) dans laquelle se forme le brouillard.

5. Nébuliseur selon la revendication 4, caractérisé par le fait que ledit conduit de sortie à l'air libre (80) est branché sur ledit conduit d'amenée (79), en aval du branchement précité dudit conduit d'accès (64).

6. Nébuliseur selon l'une des revendications 2 et 3, caractérisé par le fait que lesdits moyens de prise de pression (36, 37) sont disposés en amont de la valve d'entrée anti-retour (31) dudit conduit d'entrée (23).

7. Nébuliseur selon l'une des revendications 4 et 5, caractérisé par le fait que lesdits moyens de prise de pression (72) sont disposés en amont du branchement précité dudit conduit d'accès (64).

8. Nébuliseur selon l'une des revendications 2 et 3, caractérisé par le fait que ladite chambre est formée dans une cuve (6) fermée par un couvercle (13) sur lequel sont branchés lesdits conduits d'entrée (23) et d'amenée (29).

9. Nébuliseur selon l'une des revendications 4 et 5, caractérisé par le fait que ladite chambre (47) est formée dans une cuve munie d'un couvercle (46) sur lequel est branché ledit conduit d'accès (64).

10. Nébuliseur selon l'une quelconque des revendications précédentes, caractérisé par le fait que lesdits moyens de prise de pression comprennent une tubulure (36) branchée sur lesdits moyens de transport, au fond de laquelle est disposé un capteur de pression (37).

11. Nébuliseur selon l'une quelconque des revendications précédentes, caractérisé par le fait que lesdits moyens (39) pour délivrer un signal de signalisation ou d'avertissement déterminent deux seuils de niveau du signal de signalisation ou d'avertissement.

12. Nébuliseur selon l'une quelconque des revendications précédentes, caractérisé par le fait que lesdits moyens pour délivrer un signal de signalisation ou d'avertissement comprennent des moyens visuels (39).

13. Nébuliseur selon l'une quelconque des revendications précédentes, caractérisé par le fait que lesdits moyens pour délivrer un signal de signalisation ou d'avertissement comprennent des diodes électroluminescentes (39) d'au moins deux couleurs permettant de distinguer ledit seuil de niveau et des moyens pour alimenter sélectivement ces diodes en fonction de la valeur du signal fourni par lesdits moyens de prise de pression.

14. Nébuliseur selon l'une quelconque des revendications précédentes, caractérisé par le fait que le niveau des signaux desdits moyens (39) de signalisation ou d'avertissement est réglable.
